# FASCICULE DE BREVET EUROPEEN

(11) **EP 1 465 737 B1**
(45) Date de publication et mention de la délivrance du brevet: **25.02.2009**
(21) Numéro de dépôt: 02801146.8
(22) Date de dépôt: 17.12.2002
(51) Int. Cl.: B05B 11/04

(54) **DISTRIBUTEUR DE PRODUIT FLUIDE**
FLUIDPRODUKTSPENDER
FLUID PRODUCT DISPENSER

(30) Priorité: 20.12.2001 FR 0116553
(43) Date de publication de la demande: 13.10.2004
(73) Titulaire: Valois SAS, 27110 Le Neubourg (FR)
(72) Inventeur: GARCIA, Firmin, F-27000 Evreux (FR); LECOUTRE, Jean-Paul, F-27160 Breteuil sur Iton (FR); MILIAN, Alex, F-27160 Breteuil sur Iton (FR)
(74) Mandataire: CAPRI
(86) Numéro de dépôt international: PCT/FR2002/004407
(87) Numéro de publication internationale: WO 2003/053592

(56) Documents cités:
- FR-A- 1 525 560
- FR-A- 2 610 302
- US-A- 4 432 496

## Description

La présente invention concerne un distributeur de produit fluide comprenant un réservoir contenant du produit fluide et un gaz, ledit réservoir comprenant une ouverture, un fond et une paroi d'actionnement apte à être déplacée pour diminuer le volume du réservoir, un orifice de distribution à travers lequel le produit fluide est distribué à chaque actionnement de la paroi d'actionnement, et une pièce de matière poreuse apte à s'imbiber de produit fluide par capillarité étant disposée à proximité directe de l'orifice de distribution avec une extrémité de décharge. Un tel distributeur est notamment décrit dans les documents US-4 858 831 et FR-2 781 770. Dans les deux documents, il s'agit de distributeur se présentant sous la forme d'échantillon de produit fluide dans le domaine de la cosmétique, de la parfumerie ou encore de la pharmacie.

Dans l'art antérieur précité, la pièce de matière poreuse s'étend dans le réservoir de produit fluide de sorte qu'elle peut librement s'imbiber de produit fluide par capillarité. La pièce de matière poreuse se charge ainsi d'une certaine quantité qui est dépendante de sa capacité d'absorption. Après plusieurs actionnements de la paroi, la pièce de matière poreuse se vide du produit fluide qu'elle imbibait. En fonction de l'orientation du distributeur, il peut arriver que la pièce de matière poreuse ne soit pas en contact du produit fluide stocké à l'intérieur du réservoir. Dans ce cas, après un certain nombre d'actionnement de la paroi, la pièce de matière poreuse est vide de produit fluide, de sorte que le distributeur n'expulse pratiquement plus que de l'air et une quantité presque infime de produit fluide.

Le document FR-1525 560 décrit un distributeur comprenant un réservoir à parois déformables, un orifice de distribution, une pièce de matière spongieuse ainsi qu'un tube-plongeur. Il est prévu une chambre intermédiaire entre la pièce de matière spongieuse et l'orifice de distribution.

La présence d'une chambre entre la pièce de matière spongieuse et l'orifice de distribution conduit dans certains cas à une accumulation de produit fluide dans cette chambre, de sorte que l'on obtient un jet de produit fluide et non pas une pulvérisation biphasique.

La présente invention est définie par la revendication 1 dont préambule est connu de FR-A-2 510 302 où on utilise le tube-plongeur pour alimenter la pièce de matière poreuse qui sert en quelque sorte de réservoir tampon disposé à proximité directe de l'orifice de distribution.

Selon la présente invention, au moins un passage fait communiquer la pièce de matière poreuse avec le réservoir sans passer par le tube-plongeur, de sorte que du gaz et du produit fluide sont simultanément chassés à travers la pièce de matière poreuse à chaque actionnement de la paroi d'actionnement. Ceci est vrai pratiquement dans n'importe quelle position du distributeur : en effet, lorsque celui-ci est maintenu debout avec son orifice de distribution orienté vers le haut, le produit fluide est stocké dans le fond du réservoir et c'est alors le tube-plongeur qui achemine le produit fluide vers la pièce de matière poreuse alors que le passage achemine du gaz avantageusement de l'air à la même pièce de matière poreuse. A l'inverse, lorsque l'orifice de distribution est dirigé vers le bas, le produit fluide est stocké à proximité de son ouverture de sorte que le passage fait communiquer du produit fluide à la pièce de matière poreuse, alors que le tube-plongeur permet d'acheminer de l'air puisque son extrémité libre n'est pas plongée dans le produit fluide. Le passage s'étend partiellement le long du tube-plongeur.

Selon l'invention, l'orifice de distribution est formé par un embout monté sur l'ouverture du réservoir, ledit embout formant un logement de réception dans lequel la pièce de matière poreuse est engagée. L'embout forme un manchon de fixation avec lequel le tube-plongeur est en prise avec son extrémité de raccordement. Avantageusement, l'extrémité de raccordement du tube-plongeur est en contact de la pièce de matière poreuse. Avantageusement, le manchon de fixation peut être en prise dans l'ouverture du réservoir. De préférence, ledit au moins un passage est formé à l'intérieur du manchon et s'étend jusqu'au niveau du logement de réception.

Une forme de réalisation pratique peut prévoir que l'embout comprend un corps définissant une extrémité de distribution formant l'orifice de distribution et une extrémité opposée de fixation pourvue d'un manchon de fixation auquel le tube-plongeur est raccordé, ledit corps formant intérieurement le logement de réception pour la pièce de matière poreuse, ledit logement et ledit manchon s'étendant dans le prolongement l'un de l'autre de sorte que l'extrémité de raccordement du tube-plongeur est apte à venir en contact de la pièce de matière poreuse.

L'invention sera maintenant plus amplement décrite en référence aux dessins joints donnant à titre d'exemple non limitatif un mode de réalisation de l'invention.

Sur les figures :
- la figure 1 est une vue en section transversale verticale de profil d'un distributeur de produit fluide selon la présente invention,
- la figure 2 est une vue similaire à celle de la figure 1 mais de face, et
- la figure 3 est une vue agrandie de la partie supérieure du distributeur de la figure 1.

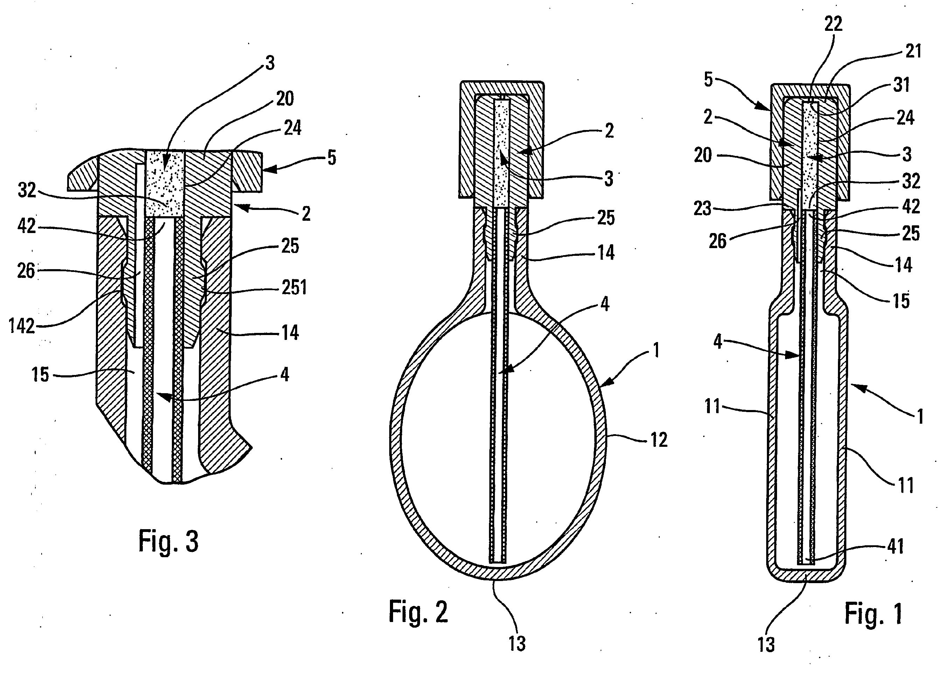

Le distributeur représenté dans son intégralité sur les figures 1 et 2 comprend un réservoir 1, un embout de distribution 2, une pièce de matière poreuse 3, un tube-plongeur 4 et optionnellement un capuchon 5.

Le réservoir 1 comprend un fond 13 et des parois latérales 11 et 12. On peut remarquer que les parois latérales 12 sont courbes ou arrondies et se rejoignent dans leur partie inférieure pour former la paroi de fond 13. Quant aux deux autres parois latérales 11, au moins une d'entre elles forme une paroi d'actionnement enfonçable c'est à dire déformable de manière à faire varier le volume interne du réservoir. Ainsi, l'utilisateur peut à l'aide d'un doigt, avantageusement le pouce, appuyer sur la paroi d'actionnement 11 pour l'enfoncer, c'est à dire la déplacer en direction de l'autre paroi latérale 11, ce qui a pour effet de réduire le volume interne du réservoir. Le réservoir 1 forme en outre un col 14 définissant une ouverture 15 qui permet de faire communiquer l'intérieur du réservoir avec l'extérieur. Avantageusement, la paroi interne du col 14 peut être pourvue d'un évidement 142 dont la fonction de fixation ou de maintien sera décrite ci-après.

La forme spécifique du réservoir ici décrite ne doit pas être considérée comme limitative. Au contraire, on peut imaginer un réservoir ayant une forme quelconque mais comprenant au moins une paroi d'actionnement enfonçable ou déformable.

Selon la forme de réalisation de l'invention, l'embout 2 est monté sur, ou plus précisément dans l'ouverture 15 formée par le col 14 du réservoir 1. L'embout 2 définit un orifice de distribution 22. Ainsi, le produit fluide stocké à l'intérieur du réservoir 1 peut être refoulé à travers l'orifice de distribution 22 formé par l'embout 2 lorsque l'on appuie sur la paroi d'actionnement 11 de manière à réduire le volume interne du réservoir. Selon l'invention, le réservoir contient une faible quantité de produit fluide, le restant du volume du réservoir étant rempli par un gaz, de préférence de l'air. De cette manière, c'est un mélange de produit fluide et d'air qui est refoulé à travers l'orifice de distribution 22 lorsque l'on appuie sur la paroi d'actionnement 1. En résultat, on obtient un jet pulvérisé biphasique de produit fluide et d'air.

L'embout 2 peut se présenter, comme représenté sur les figures, sous la forme d'un corps 20 présentant une extrémité de distribution 21 dans laquelle est formé l'orifice de distribution 22 et une extrémité opposée de fixation 23 à partir de laquelle s'étend un manchon de fixation 25 qui est engagé à l'intérieur de l'ouverture 15 formée par le col 14 du réservoir. En se référant à la figure 3, on peut voir que le manchon 25 forme extérieurement un ou plusieurs profil(s) d'encliquetage 251 destiné(s) à venir se loger à l'intérieur de l'évidement 142 formé dans la paroi interne du col 14. La mise en place de l'embout 2 sur le réservoir 1 est donc très simple : il suffit d'engager le manchon 25 dans l'ouverture 15 du col 14 et d'appliquer une pression suffisante sur l'embout 2 de manière à le faire pénétrer en force dans l'ouverture 15. En position finale représentée de manière agrandie sur la figure 3, le ou les profil(s) d'encliquetage 251 sont encliquetés dans l'évidement 142. Ceci assure une fixation parfaitement solide de l'embout 2 sur le réservoir 1.

Selon l'invention, l'embout 2 forme un logement interne de réception 24 qui s'étend entre le manchon 25 et l'orifice de distribution 22. Ce logement de réception 24 contient selon l'invention une pièce de matière poreuse 3 adaptée à s'imbiber de produit fluide par capillarité. Cette pièce de matière poreuse peut être réalisée à partir d'une mousse ou d'une fibre qui définit un réseau interne de canaux de section réduite pour conférer la caractéristique d'absorption par capillarité. La pièce de matière poreuse 3 comprend une extrémité de décharge 31 située de manière adjacente à, ou à proximité directe, de l'orifice de distribution 22 et une extrémité d'alimentation 32 qui est située sensiblement au niveau où le manchon de raccordement 25 se raccorde au corps 20. Bien entendu, l'extrémité d'alimentation 32 peut également être située directement à l'intérieur du manchon 25 ou au contraire s'arrêter dans le corps 20. De préférence, l'extrémité de décharge 31 est en contact direct de l'extrémité de distribution 21 où est formé l'orifice de distribution, de sorte que l'extrémité de décharge de la pièce de matière poreuse est en contact de l'orifice de distribution.

Selon l'invention, le tube-plongeur 4 comprend une extrémité libre 41 située à proximité du fond 13 du réservoir 1 et une extrémité opposée de raccordement 42 en prise à l'intérieur du manchon de fixation 25. Le tube-plongeur 4 peut être simplement emmanché à force dans le manchon de fixation 25. Avantageusement, l'extrémité de raccordement 42 du tube-plongeur 4 peut venir en contact avec l'extrémité d'alimentation 32 de la pièce de matière poreuse 3 comme on peut le voir clairement sur la figure 3. De cette manière, du produit fluide stocké au niveau du fond 13 du réservoir 1 peut être alimenté jusqu'à la pièce de matière poreuse 3 à travers le tube-plongeur 4 en appuyant sur la paroi d'actionnement 11. A l'inverse, si le distributeur est maintenu sensiblement à l'envers avec son extrémité libre 41 s'étendant hors du produit fluide, la pièce de matière poreuse 3 peut être alimentée en air à travers le tube-plongeur 4 par appui sur la paroi d'actionnement 11. Etant donné qu'un distributeur est le plus souvent utilisé avec son orifice de distribution 22 situé au-dessus du fond 13 du réservoir, le tube-plongeur 4 garantit que la pièce de matière poreuse 3 sera toujours alimentée en produit fluide. On garantit ainsi une distribution du produit fluide à travers l'orifice de distribution 22.

Selon une autre caractéristique intéressante de l'invention, le distributeur peut également comprendre un ou plusieurs passages 26 qui font communiquer directement la pièce de matière poreuse avec le réservoir 1 sans passer par le tube-plongeur 4. Dans la forme de réalisation utilisée pour illustrer la présente invention, ce passage 26 est formé par l'embout 2 sous la forme d'une saignée ou d'une rainure qui s'étend à l'intérieur du manchon 25 jusqu'au niveau du corps 20. Bien entendu, on peut prévoir plusieurs passages 26 par exemple répartis autour du tube-plongeur 4 à l'intérieur du manchon 25. Ce ou ces passages 26 permettent d'alimenter la pièce de matière poreuse 3 en air lorsque le distributeur est maintenu avec son orifice de distribution 22 au-dessus de son fond 13. A l'inverse, lorsque le distributeur est maintenu à l'envers, le ou les passages 26 permettent une alimentation directe de produit fluide à la pièce de matière poreuse 3. En même temps, le tube-plongeur 4 assure une alimentation en air.

L'utilisation d'un ou de plusieurs passages 26 dans un distributeur comprenant un tube-plongeur associé à une pièce de matière poreuse 3 permet d'assurer que la pièce de matière poreuse 3 est toujours alimentée à la fois en produit fluide et en air, et ceci dans n'importe quelle position. Le tube-plongeur peut alimenter du produit fluide et le passage de l'air ou inversement, le tube-plongeur peut alimenter de l'air et le passage du produit fluide.

Optionnellement, l'embout 3 peut être coiffé d'un capuchon 5 permettant de masquer ou d'obturer l'orifice de distribution 22.

## Revendications

1. Distributeur de produit fluide comprenant :
- un réservoir (1) contenant du produit fluide et un gaz, ledit réservoir comprenant une ouverture (15), un fond (13) et une paroi d'actionnement (11) apte à être déplacée pour diminuer le volume du réservoir,
- un orifice de distribution (22) à travers lequel le produit fluide est distribué à chaque actionnement de la paroi d'actionnement, l'orifice de distribution (22) étant formé par un embout (2) monté sur l'ouverture (15) du réservoir (1), ledit embout (2) formant un logement de réception (24), l'embout (2) formant un manchon de fixation (25) avec lequel un tube-plongeur (4) est en prise avec son extrémité de raccordement (42),
- le tube-plongeur (4) s'étendant avec une extrémité libre (41) dans le réservoir (1) sensiblement jusqu'à proximité de son fond (13), ledit tube-plongeur (4) comprenant une extrémité opposée de raccordement (42) apte à alimenter du produit fluide à une pièce de matière poreuse (13), et
- la pièce de matière poreuse (3) apte à s'imbiber de produit fluide par capillarité étant disposée dans le logement de réception (24) entre le tube-plongeur (4) et l'orifice de distribution (22), la pièce de matière poreuse (3) comprenant une extrémité de décharge (31) à proximité directe de l'orifice de distribution (22),
**caractérisé en ce qu'**au moins un passage (26) fait communiquer la pièce de matière poreuse (3) avec le réservoir (1) sans passer par le tube-plongeur (4), de sorte que du gaz et du produit fluide sont simultanément chassés à travers la matière de la pièce de matière poreuse (3) à chaque actionnement de la paroi d'actionnement (11), le passage (26) s'étendant partiellement le long et à l'extérieur du tube-plongeur (4).

2. Distributeur selon la revendication 1, dans lequel l'extrémité de raccordement (42) du tube-plongeur est en contact de la pièce de matière poreuse (3).

3. Distributeur selon la revendication 1 ou 2, dans lequel le manchon de fixation (25) est en prise dans l'ouverture (15) du réservoir.

4. Distributeur selon l'une quelconque des revendications 1 à 3, dans lequel le au moins un passage (26) est formé à l'intérieur du manchon (25) et s'étend jusqu'au niveau du logement de réception (24).

5. Distributeur selon la revendication 4, dans lequel le passage (26) se présente sous la forme d'une saignée qui s'étend à l'intérieur du manchon (25) recevant le tube-plongeur.

6. Distributeur selon l'une quelconque des revendications 1 à 5, dans lequel l'embout (2) comprend un corps (20) définissant une extrémité de distribution (21) formant l'orifice de distribution (22) et une extrémité opposée de fixation (23) pourvue du manchon de fixation (25) auquel le tube-plongeur (4) est raccordé, ledit corps (20) formant intérieurement le logement de réception (24) pour la pièce de matière poreuse (3), ledit logement et ledit manchon s'étendant dans le prolongement l'un de l'autre de sorte que l'extrémité de raccordement (42) du tube-plongeur est apte à venir en contact de la pièce de matière poreuse (3).

7. Distributeur selon l'une quelconque des revendications précédentes, dans lequel l'extrémité de décharge (31) de la pièce de matière poreuse (3) est en contact de l'orifice de distribution (22).

## Claims

1. Fluid product dispenser comprising:
- a reservoir (1) containing the fluid product and a gas, the said reservoir comprising an opening (15), a bottom (13), and an actuation wall (11) that can be moved to reduce the volume of the reservoir,
- a dispensing orifice (22) through which the fluid product is distributed every time that the actuation wall is actuated, the dispensing orifice (22) being formed by an end piece (2) installed on the opening (15) of reservoir (1), the said end piece (2) forming a reception housing (24), the end piece (2) forming an attachment sleeve (25) with which the plunger tube (4) comes into contact with its connection end (42),
- the plunger tube (4) extending with a free end (41) into the reservoir (1) almost to its bottom (13), the said plunger tube (4) comprising an opposite connection end (42) through which fluid product is supplied to a porous material part (3), and
- the porous material part (3) able to absorb fluid product by capillarity being placed in the reception housing (24) between the plunger tube (4) and the dispensing orifice (22), the porous material part (3) comprising a discharge end (31) located in the immediate vicinity of the dispensing orifice (22),
**characterized in that** at least one passage (26) forms a passageway between the porous material part (3) and the reservoir (1) without passing through the plunger tube (4), such that the gas and the fluid product are simultaneously pushed through the material of the porous material part (3) every time that the actuation wall (11) is pressed, the passage (26) extends partially along and outside the plunger tube (4).

2. Dispenser according to claim 1, wherein the connection end (42) of the plunger tube is in contact with the porous material part (3).

3. Dispenser according to claim 1 or 2, wherein the attachment sleeve (25) is gripped in the reservoir opening (15).

4. Dispenser according to claim 1, 2 or 3, wherein the at least one passage (26) is formed inside the sleeve (25) and extends as far as the reception housing (24).

5. Dispenser according to claim 4, wherein the passage (26) has the shape of a groove that extends inside the sleeve (25) which receives the plunger tube.

6. Dispenser according to any preceding claim, wherein the end piece (2) comprises a body (20) defining a dispensing end (21) forming the dispensing orifice (22) and an opposite attachment end (23) provided with the attachment sleeve (25) to which the plunger tube (4) is connected, the inside of the said body (20) forming the reception housing (24) for the porous material part (3), the said housing and the said sleeve being aligned with each other such that the connection end (42) of the plunger tube can come into contact with the porous material part (3).

7. Dispenser according to any one preceding claim, wherein the discharge end (31) is in contact with the dispensing orifice (22).

## Patentansprüche

1. Spender für ein flüssiges Produkt, aufweisend:
einen Behälter (1), der ein flüssiges Produkt und ein Gas enthält, wobei der Behälter eine Öffnung (15), einen Boden (13) und eine Betätigungswandung (11) aufweist, die versetzt werden kann, um das Volumen des Behälters zu verkleinern,
eine Ausgabeöffnung (22), durch die das flüssige Produkt bei jeder Betätigung der Betätigungswandung ausgegeben wird, wobei die Ausgabeöffnung (22) durch einen Ansatz (2) gebildet ist, der auf der Öffnung (15) des Behälters (1) angebracht ist, wobei der Ansatz (2) einen Aufnahmesitz (24) bildet, wobei der Ansatz (2) eine Befestigungsmuffe (25) bildet, mit der ein Tauchrohr (4) mit seinem Verbindungsende (42) im Eingriff ist,
wobei sich das Tauchrohr (4) mit einem freien Ende (41) in den Behälter (1) etwa bis nahe seines Bodens (13) erstreckt, wobei das Tauchrohr (4) ein entgegengesetztes Verbindungsende (42) aufweist, das dazu geeignet ist, einem Teil aus porösem Material (3) ein flüssiges Produkt zuzuführen, und
das Teil aus porösem Material (3) dazu geeignet ist, durch Kapillarwirkung ein flüssiges Produkt aufzusaugen, wenn es in dem Aufnahmesitz (24) zwischen dem Tauchrohr (4) und der Ausgabeöffnung (22) angeordnet ist, wobei das Teil aus porösem Material (3) ein Auslassende (31) in unmittelbarer Nähe zur Ausgabeöffnung (22) aufweist,
**dadurch gekennzeichnet, dass** mindestens ein Durchlass (26) eine Verbindung zwischen dem Teil aus porösem Material (3) und dem Behälter (1) herstellt, ohne dabei das Tauchrohr (4) zu passieren, so dass bei jeder Betätigung der Betätigungswandung (11) Gas und flüssiges Produkt gleichzeitig durch das Material des Teils aus porösem Material (3) ausgetrieben werden, wobei sich der Durchlass (26) teilweise entlang und außerhalb des Tauchrohrs (4) erstreckt.

2. Spender nach Anspruch 1, wobei das Verbindungsende (42) des Tauchrohrs mit dem Teil aus porösem Material (3) in Kontakt steht.

3. Spender nach Anspruch 1 oder 2, wobei die Befestigungsmuffe (25) in der Öffnung (15) des Behälters im Eingriff ist.

4. Spender nach einem der Ansprüche 1 bis 3, wobei der mindestens eine Durchlass (26) im Inneren der Muffe (25) gebildet ist und sich bis zur Höhe des Aufnahmesitzes (24) erstreckt.

5. Spender nach Anspruch 4, wobei sich der Durchlass (26) in Form einer Drainung darstellt, die sich im Inneren der Muffe (25) erstreckt, die das Tauchrohr aufnimmt.

6. Spender nach einem der Ansprüche 1 bis 5, wobei der Ansatz (2) einen Körper (20), der ein Ausgabeende (21) begrenzt, das die Ausgabeöffnung (22) bildet, und ein entgegengesetztes Befestigungsende (23) aufweist, das mit der Befestigungsmuffe (25) versehen ist, mit der das Tauchrohr (4) verbunden ist, wobei der Körper (20) im Inneren den Aufnahmesitz (24) für das Teil aus porösem Material (3) bildet, wobei sich der Sitz und die Muffe jeweils in der Verlängerung zueinander so erstrecken, dass das Verbindungsende (42) des Tauchrohrs dazu geeignet ist, mit dem Teil aus porösem Material (3) in Kontakt zu treten.

7. Spender nach einem der vorhergehenden Ansprüche, wobei das Auslassende (31) des Teils aus porösem Material (3) mit der Ausgabeöffnung (22) in Kontakt ist.
